# EUROPEAN PATENT SPECIFICATION

(11) **EP 1 273 587 B1**
(45) Date of publication and mention of the grant of the patent: **10.06.2009**
(21) Application number: 01919924.9
(22) Date of filing: 13.04.2001
(51) Int. Cl.: C07D 501/04, C07D 501/22

(54) **PROCESS FOR THE PREPARATION OF A 3-VINYLCEPHEM COMPOUND**
VERFAHREN ZUR HERSTELLUNG EINER 3-VINYLCEPHEM-VERBINDUNG
PROCEDE DE PREPARATION D'UN COMPOSE 3-VINYLCEPHEME

(30) Priority: 13.04.2000 JP 2000111448
(43) Date of publication of application: 08.01.2003
(73) Proprietor: OTSUKA KAGAKU KABUSHIKI KAISHA, Osaka-shi, Osaka-fu 540-0021 (JP)
(72) Inventor: KAMEYAMA, Yutaka, c/o OTSUKA KAGAKU K.K., Tokushima-shi, Tokushima 771-0193 (JP); FUKAE, Kazuhiro, c/o OTSUKA KAGAKU K.K., Tokushima-shi, Tokushima 771-0193 (JP)
(74) Representative: Barz, Peter
(86) International application number: PCT/JP2001/003182
(87) International publication number: WO 2001/079211

(56) References cited:
- EP-A2- 0 105 459
- WO-A-97/24358
- WO-A-03/091261
- WO-A1-96/26943

## Description

### TECHNICAL FIELD

The present invention relates to a process for preparing a cefdinir compound which is widely used as an antibiotic for oral application.

### BACKGROUND ART

The cefdinir compound is mostly prepared in the form wherein at least one of amino, oxime hydroxyl and carboxyl groups is protected. In the process for preparing the compound, a reaction for removing the protection is carried out in the final step, giving (6R, 7R)-3-vinyl-8-oxo-7β-[(z)-2-(2-amino-4-thiazolyl)-2-hydroxyiminoacetamide]-1-aza-5-thiabicyclo[4,2,0]octane-2-carboxylic acid (cefdinir). However, a decisive method has not been established for deprotection of cefdinir compound having various functional groups in the molecule. For example, JP-B-1-49273 discloses a reaction for deprotection of a compound of the formula (1) wherein R¹=R²=H, R³=CHPh₂ in anisole/acetic acid in the presence of etherate of boron trifluoride. The disclosed method can not be industrially utilized since it produces the contemplated compound in a low yield of 35% and requires a large amount of a boron trifluoride compound which is hazardous. JP-A-62-294687 describes a method for deprotection of cephem antibiotics which is extensively conducted, more specifically, a deprotection method using trifluoroacetic acid in the presence of anisole. The method, however, requires a large amount of trifluoroacetic acid which is difficult to industrially use for the reason that the acid is volatile, cumbersome to handle and expensive. In addition, the yield is as low as 28%. Therefore, the method is far from industrially proper.

WO 07/24358 discloses a process for preparing cefdinir which employs a specific cefdinir intermediate in salt form as a starting material, the trityl protecting group of which is removed in the presence of an acid.

Methods are known for deprotection of protected group of carboxylic acid although not for preparing cefdinir. These methods include a method using 99% formic acid as a solvent [Chem. Pharm. Bull., 30, 4545 (1982)], a method wherein carboxylic acid ester is reacted with aluminum chloride in the presence of anisole [Tetrahedron Lett., 2793 (1979)], and a method using phenols [J. Org. Chem., 56, 3633 (1991)]. The method using formic acid needs expensive 99% formic acid as a solvent in an excessively large amount and gives a carboxylic compound in a very low yield since β -lactam derivative which is instable to an acid decomposes in the procedure for recovery and reuse. The method using aluminum chloride in the presence of anisole is not applicable to the preparation of cefdinir because of high acidity of aluminum chloride. The method using phenols is unable to carry out a reaction in a manner to result in a high yield because cefdinir is instable under highly acidic conditions as is the case with use of formic acid or trifluoroacetic acid in a large amount. All of these reactions eventually give cefdinir wherein the oxime group is made into hydroxyl group so that syn/anti isomerization proceeds in a large amount of protonic acid and strong Lewis acid, resulting in increase of improper impurities. Thus these deprotection methods can not be employed.

It has been very difficult heretofore, as described above, to prepare the contemplated cefdinir compound with a high selectivity in a high yield since the deprotection reaction is conducted by usual acid hydrolysis in a β-lactam compound. Thus, it is desired to develop an industrially inexpensive and efficient deprotection method.

An object of the present invention is to provide a novel technique capable of efficiently preparing 3-vinyl-cephem compound of the formula (2) from a protected 3-vinyl-cephem derivative of the formula (1) without use of an expensive reagent.

### DISCLOSURE OF THE INVENTION

The present invention provides a process for preparing 3-vinyl-cephem compound of the formula (2), the process comprising the step of treating a protected 3-vinyl-cephem derivative of the formula (1) in an organic solvent in the presence of perhalogenated acid and an organic protonic acid 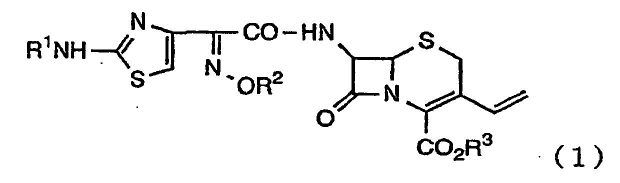 wherein each of R¹, R² and R³ is a hydrogen atom or arylmethyl group optionally having a substituent, provided that R¹, R² and R³ can not be a hydrogen atom at the same time.

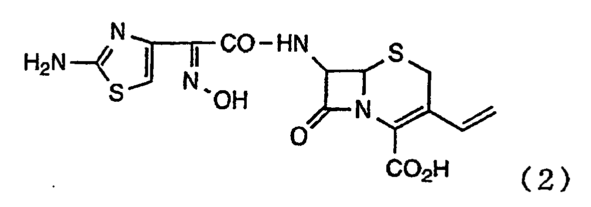

In the present invention, hydrogen bonding of an organic protonic acid is conducted in an organic solvent, the acid being weak against an amide group and amino group in the skeleton of the raw material, and only a required amount of strong perhalogenated acid is used in order to efficiently bring about a reaction for deprotection of cefdinir compound which is instable to an acid. Thereby it becomes possible to prepare a highly stable cefdinir compound with high efficiency. The cefdinir compound can stably exist in the reaction system because the reaction employs only a required minimum amount of strong perhalogenated acid which can contribute to the reaction. This process has another feature. Since the reaction need not use a large amount of acid, the desired compound can be isolated from the reaction product by merely extracting the compound dissolved in the organic solvent using a required amount of a base corresponding to the amount of the acid used. Thus a process capable of preparing the contemplated compound industrially easily and inexpensively has been successfully established according to the invention.

Examples of the arylmethyl group optionally having a substituent which group is represented by R¹, R² and R³, respectively are benzyl, diphenylmethyl, trityl, anisylmethyl and naphthylmethyl which may have a substituent. Examples of the substituent are hydroxy, methyl, ethyl, tert-butyl and like lower alkyl groups having 1 to 4 carbon atoms, and methoxy, ethoxy and like lower alkoxy groups having 1 to 4 carbon atoms. The diphenylmethyl includes the groups of the type wherein a substituted or unsubstituted phenyl group is bonded in the molecule via methylene chain or hetero atom. Specific examples of diphenylmethyl groups are benzyl, p-methoxybenzyl, diphenylmethyl, trityl, 3,4,5-trimethoxybenzyl, 3,5-dimethoxy-4-hydroxybenzyl, 2,4,6-trimethylbenzyl and ditolylmethyl.

Examples of organic protonic acids which can be used in the invention include preferably those having pKa of 3 to 5 such as formic acid, acetic acid, chloroacetic acid, propionic acid, 2-ethylhexanoic acid and like substituted or unsubstituted lower alkylcarboxylic acid, benzoic acid, toluic acid and like substituted or unsubstituted aromatic carboxylic acids which can be widely used.

The amount of the organic protonic acid used is 1 to 20 mole equivalents, preferably 2.5 to 10 mole equivalents and more preferably 3 to 5 mole equivalents, per mole equivalent of the compound of the formula (1).

Examples of the perhalogenated acid are perchloric acid, periodic acid and perbromic acid. The amount of the perhalogenated acid used is equal to a catalytic amount, and is preferably 0.1 to 5 mole equivalents per mole equivalent of the compound of the formula (1).

As to the concentration of the perhalogenated acid, 60% perhalogenated acid which is commercially available can be used as it is. Perhalogenated acid is usable when diluted to 10 to 50% with the reaction system.

Examples of the organic solvent which can be used in the invention are methyl formate, ethyl formate, propyl formate, butyl formate, methyl acetate, ethyl acetate, propyl acetate, butyl acetate, methyl propionate, ethyl propionate and like lower alkyl esters of lower carboxylic acids, acetone, methyl ethyl ketone, methyl propyl ketone, methyl butyl ketone, methyl isobutyl ketone, diethyl ketone and like ketones, acetonitrile, propionitrile, butyronitrile, isobutyronitrile, valeronitrile and like nitriles, benzene, toluene, xylene, chlorobenzene, anisole and like substituted or unsubstituted aromatic hydrocarbons, dichloromethane, chloroform, dichloroethane, trichloroethane, dibromoethane, propylenedichloride, carbon tetrachloride and like hydrocarbon halides, pentane, hexane, heptane, octane and aliphatic hydrocarbons, cyclopentane, cyclohexane, cycloheptane, cyclooctane and like cycloalkanes. Preferred solvents are benzene, toluene, xylene, dichloromethane, chloroform and dichloroethane. These organic solvents can be used either alone or in combination. These solvents may contain water when so required. The solvents may be used in an amount of about 2 to about 200 liters, preferably about 3 to about 100 liters, per kilogram of the compound of the formula (1). The reaction may be conducted at a temperature of -20 to 100°C, preferably 0 to 50°C.

The compound of the formula (2) can be obtained as a substantially pure product by usual extraction or crystallization after completion of reaction, and of course, can be purified by other methods.

### BEST MODE OF CARRYING OUT THE INVENTION

The present invention will be described in more detail with reference to the following examples to which the invention, however, is not limited.

### Example 1

Dissolved in 10 ml of methylene chloride was 1 g of a compound (1a), namely the compound of the formula (1) wherein R¹ is a hydrogen atom, R² is a trityl group and R³ is a hydrogen atom. To the solution were added 0.18 ml (3 equivalents) of 98% (w/w) formic acid, and 0.16 ml (1.6 equivalents) of 60% (w/w) of perchloric acid. Then the mixture was reacted at 30°C for 1 hour. To the reaction mixture was added 7 ml of a saturated aqueous solution of sodium bicarbonate to extract the desired product. 2N hydrochloric acid was added to the obtained aqueous layer to adjust a pH to 3.0. The mixture was cooled to 0 to 3°C. One hour later, the precipitated crystals were subjected to suction filtration and dried under reduced pressure, giving 0.59 g (yield 95%) of the contemplated cefdinir compound of the formula (2).
1H NMR (DMSO-d₆) 3.32(s, 1H), 3.53(d, J=18Hz, 1H), 3.81(d, J=18Hz, 1H), 5.16(d, J=4.8Hz, 1H), 5.29(d, J=11.7Hz, 1H), 5.56(d, J=17.1Hz, 1H), 5.76(dd, J=4.8, 8.1Hz, 1H), 6.64(s, 1H), 6.89(dd, J=11.7, 17.1Hz, 1H), 7.11(s, 2H), 9.47(d, J=8.1Hz, 1H). 11.3(s,1H).

### Example 2

A reaction was carried out in the same manner as in Example 1 using 2 dimethylacetamide-coordination crystals of p-toluene sulfonate of compound (1a), whereby a cefdinir compound of the formula (2) was produced in a yield of 96%. The 1H NMR data of the obtained cefdinir compound were identical with those of the compound produced in Example 1.

### Examples 3 to 8

The same reaction as in Example 1 was conducted with the exception of using different solvents and adjusting the reaction time according to the solvent used. The results of the reaction are shown in Table 1.

**Table 1**

| Example | organic solvent | reaction time(hr) | yield (%) |
|---|---|---|---|
| 3 | chloroform | 1 | 95 |
| 4 | benzene | 1 | 94 |
| 5 | toluene | 1 | 94 |
| 6 | xylene | 1 | 92 |
| 7 | ethyl acetate | 4 | 90 |
| 8 | butyl acetate | 4 | 89 |

### Examples 9 to 12

The same reaction as in Example 1 was conducted with the exception of using perchloric acid in different concentrations and adjusting the reaction time. The results of the reaction are shown in Table 2.

**Table 2**

| Example | concentration of perchloric acid (%) | reaction time (hr) | yield (%) |
|---|---|---|---|
| 9 | 45 | 1 | 96 |
| 10 | 30 | 1 | 95 |
| 11 | 20 | 1.5 | 92 |
| 12 | 10 | 6 | 87 |

### Examples 13 to 16

The same reaction as in Example 1 was conducted with the exception of using acids shown in Table 3 instead of protonic acid. The results are shown in Table 3.

**Table 3**

| Example | protonic acid | yield (%) |
|---|---|---|
| 13 | acetic acid | 95 |
| 14 | propionic acid | 93 |
| 15 | 2-ethylhexanoic acid | 86 |
| 16 | benzoic acid | 89 |

### Example 17

The same reaction as in Example 1 was conducted with the exception of using a compound of the formula (1b) wherein R¹ is a trityl group, R² is a trityl group and R³ is a hydrogen atom in place of the compound (1a), whereby cefdinir compound of the formula (2) was produced in a yield of 91%. The 1H NMR data of the obtained cefdinir compound were identical with those of the compound produced in Example 1.

### Example 18

The same reaction as in Example 1 was conducted with the exception of using a compound of the formula (lc) wherein R¹ is a hydrogen atom, R² is a trityl group and R³ is a p-methoxybenzyl group in place of the compound (1a), whereby a cefdinir compound of the formula (2) was produced in a yield of 92%. The 1H NMR data of the obtained cefdinir compound were identical with those of the compound produced in Example 1.

### Example 19

The same reaction as in Example 1 was conducted with the exception of using a compound of the formula (1d) wherein R¹ is a hydrogen atom, R² is a trityl group and R³ is a diphenylmethyl group in place of the compound (1a), whereby a cefdinir compound of the formula (2) was produced in a yield of 94%. The 1H NMR data of the obtained cefdinir compound were identical with those of the compound produced in Example 1.

### Example 20

The same reaction as in Example 1 was conducted with the exception of using a compound of the formula (1e) wherein R¹ is a trityl group, R² is a trityl group and R³ is a p-methoxybenzyl group in place of the compound (1a), whereby a cefdinir compound of the formula (2) was produced in a yield of 89%. The 1H NMR data of the obtained cefdinir compound were identical with those of the compound produced in Example 1.

### Example 21

The same reaction as in Example 1 was conducted with the exception of using a compound of the formula (1f) wherein R¹ is a trityl group, R² is a trityl group and R³ is a diphenylmethyl group in place of the compound (1a), whereby a cefdinir compound of the formula (2) was produced in a yield of 91%. The 1H NMR data of the obtained cefdinir compound were identical with those of the compound produced in Example 1.

### INDUSTRIAL APPLICABILITY

According to the present invention, a cefdinir compound which is instable to an acid can be prepared with a high purity in a high yield by carrying out in an organic solvent a sophisticated combination of hydrogen bonding with a weak acid and deprotection with a strong acid, using a combination of an organic protonic acid in an amount required for hydrogen bonding and a small amount of perhalogenated acid. The present invention can provide a process for preparing a cefdinir compound with industrially extreme ease wherein post-treatment can be simply performed due to a minimum amount of an acid used.

## Claims

1. A process for preparing 3-vinyl-cephem compound of the formula (2), the process comprising the step of treating protected 3-vinyl-cephem derivative of the formula (1) in an organic solvent in the presence of perhalogenated acid and an organic protonic acid 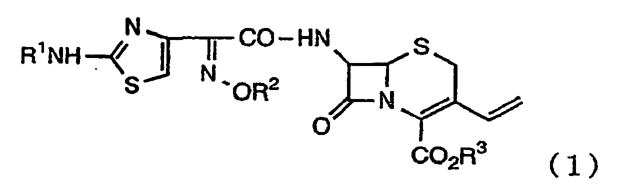 wherein each of R¹, R² and R³ is a hydrogen atom or arylmethyl group optionally having a substituent, provided that R¹, R² and R³ can not be a hydrogen atom at the same time.

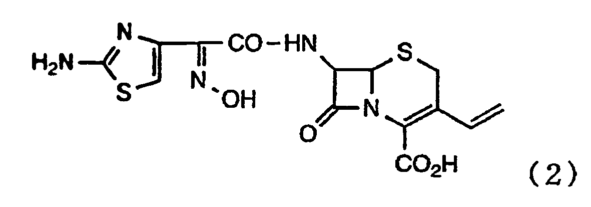

2. The process according to claim 1, wherein the reaction uses an organic protonic acid in an amount required for hydrogen bonding of protected 3-vinyl-cephem derivative of the formula (1) and a small amount of perhalogenated acid.

3. The process according to claim 2, wherein the amount of the organic protonic acid used is 1 to 20 mole equivalents per mole equivalent of the compound of the formula (1), and the amount of the perhalogenated acid used is 0.1 to 5 mole equivalents per mole equivalent of the compound of the formula (1).

4. The process according to claim 1, wherein the organic protonic acid is that having pKa of 3 to 5.

5. The process according to claim 4, wherein the organic protonic acid is formic acid, acetic acid, chloroacetic acid, propionic acid, 2-ethylhexanoic acid, benzoic acid or toluic acid.

6. The process according to claim 1, wherein the perhalogenated acid is perchloric acid, periodic acid or perbromic acid.

7. The process according to claim 1, wherein the arylmethyl group optionally having a substituent is benzyl, diphenylmethyl, trityl, anisylmethyl or naphthylmethyl.

8. The process according to claim 7, wherein the substituent is hydroxy, a lower alkyl group having 1 to 4 carbon atoms or a lower alkoxy group having 1 to 4 carbon atoms.

9. The process according to claim 7, wherein the arylmethyl group optionally having a substituent is benzyl, p-methoxybenzyl, diphenylmethyl, trityl, 3,4,5-trimethoxybenzyl, 3,5-dimethoxy-4-hydroxybenzyl, 2,4,6-trimethylbenzyl, ditolylmethyl, anisylmethyl or naphthylmethyl.

## Patentansprüche

1. Verfahren zur Herstellung einer 3-Vinylcephem-Verbindung der Formel (2), umfassend den Schritt der Behandlung eines geschützten 3-Vinylcephem-Derivats der Formel (1) in einer organischen Lösungsmittel in Gegenwart einer Perhalogensäure und einer organischen protonischen Säure 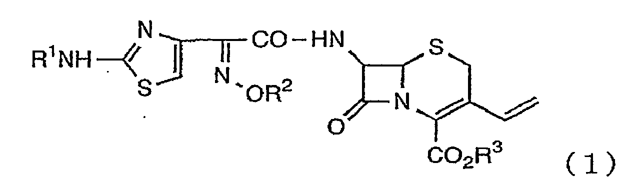 worin jedes von R¹ R² und R³ ein Wasserstoffatom oder eine Arylmethylgruppe, die gegebenenfalls einen Substituenten aufweist, bedeutet, mit der Maßgabe, dass R¹, R² und R³ nicht gleichzeitig ein Wasserstoffatom sein können.

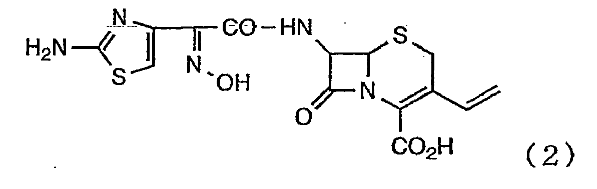

2. Verfahren nach Anspruch 1, worin die Reaktion eine organische protonische Säure in einer Menge verwendet, die zur Wasserstoffbindung des geschützten 3-Vinylcephem-Derivats der Formel (1) erforderlich ist, und eine geringe Menge von Perhalogensäure.

3. Verfahren nach Anspruch 2, worin die verwendete Menge der organischen protonischen Säure 1 bis 20 Moläquivalente pro Moläquivalent der Verbindung der Formel (1) und die verwendete Menge der Perhalogensäure 0,1 bis 5 Moläquivalente pro Moläquivalent der Verbindung der Formel (1) betragen.

4. Verfahren nach Anspruch 1, worin die organische protonische Säure einen pKa von 3 bis 5 hat.

5. Verfahren nach Anspruch 4, worin die organische protonische Säure Ameisensäure, Essigsäure, Chloressigsäure, Propionsäure, 2-Ethylhexansäure, Benzoesäure oder Toluylsäure ist.

6. Verfahren nach Anspruch 1, worin die Perhalogensäure Perchlorsäure, Periodsäure oder Perbromsäure ist.

7. Verfahren nach Anspruch 1, worin die Arylmethylgruppe, die gegebenenfalls einen Substituenten aufweist, Benzyl, Diphenylmethyl, Trityl, Anisylmethyl oder Naphthylmethyl ist.

8. Verfahren nach Anspruch 7, worin der Substituent Hydroxy, eine niedere Alkylgruppe mit 1 bis 4 Kohlenstoffatomen oder eine niedere Alkoxygruppe mit 1 bis 4 Kohlenstoffatomen ist.

9. Verfahren nach Anspruch 7, worin die Arylmethylgruppe, die gegebenenfalls einen Substituenten aufweist, Benzyl, p-Methoxybenzyl, Diphenylmethyl, Trityl, 3,4,5-Trimethoxybenzyl, 3,5-Dimethoxy-4-hydroxybenzyl, 2,4,6-Trimethylbenzyl, Ditolylmethyl, Anisylmethyl oder Naphthylmethyl ist.

## Revendications

1. Procédé de préparation du dérivé de type 3-vinyl-céphème de formule (2), lequel procédé comporte une étape consistant à traiter un dérivé de type 3-vinyl-céphème protégé, de formule (1), dans un solvant organique et en présence d'un acide perhalogéné et d'un acide organique protonique ; 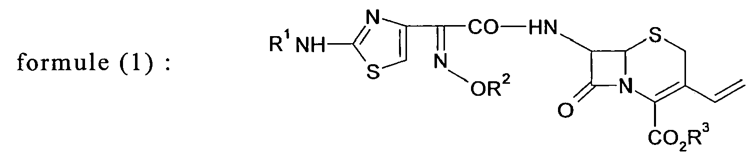 dans laquelle chacun des symboles R¹, R² et R³ représente un atome d'hydrogène ou un groupe aryl-méthyle doté, en option, d'un substituant, sous réserve que R¹ R² et R³ ne représentent pas tous en même temps des atomes d'hydrogène ;

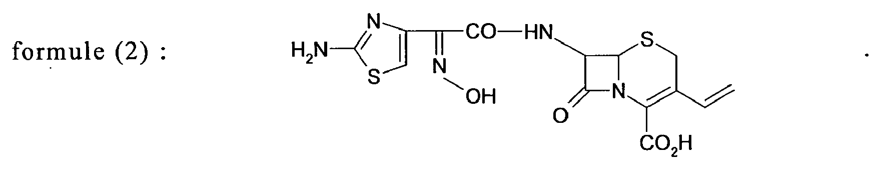

2. Procédé conforme à la revendication 1, dans lequel on emploie pour la réaction un acide organique protonique en une quantité nécessaire pour la fixation d'hydrogène par le dérivé de type 3-vinyl-céphème protégé de formule (1), ainsi qu'une petite quantité d'acide perhalogéné.

3. Procédé conforme à la revendication 2, dans lequel on emploie l'acide organique protonique en une quantité de 1 à 20 équivalents molaires par équivalent molaire du composé de formule (1), et l'acide perhalogéné en une quantité de 0,1 à 5 équivalents molaires par équivalent molaire du composé de formule (1).

4. Procédé conforme à la revendication 1, dans lequel l'acide organique protonique est un acide dont le pKa vaut de 3 à 5.

5. Procédé conforme à la revendication 4, dans lequel l'acide organique protonique est de l'acide formique, de l'acide acétique, de l'acide chloracétique, de l'acide propionique, de l'acide 2-éthyl-hexanoïque, de l'acide benzoïque, ou de l'acide toluique.

6. Procédé conforme à la revendication 1, dans lequel l'acide perhalogéné est de l'acide perchlorique, de l'acide periodique ou de l'acide perbromique.

7. Procédé conforme à la revendication 1, dans lequel le groupe aryl-méthyle doté, en option, d'un substituant est un groupe benzyle, diphényl-méthyle, trityle, anisyl-méthyle ou naphtyl-méthyle.

8. Procédé conforme à la revendication 7, dans lequel le substituant est un groupe hydroxyle, un groupe alkyle inférieur comportant 1 à 4 atomes de carbone, ou un groupe alcoxy inférieur comportant 1 à 4 atomes de carbone.

9. Procédé conforme à la revendication 7, dans lequel le groupe aryl-méthyle doté, en option, d'un substituant est un groupe benzyle, p-méthoxy-benzyle, diphényl-méthyle, trityle, 3,4,5-triméthoxy-benzyle, 3,5-diméthoxy-4-hydroxy-benzyle, 2,4,6-triméthyl-benzyle, ditolyl-méthyle, anisyl-méthyle ou naphtyl-méthyle.
